# EUROPEAN PATENT SPECIFICATION

(11) **EP 0 527 762 B1**
(45) Date of publication and mention of the grant of the patent: **14.08.1996**
(21) Application number: 91907303.1
(22) Date of filing: 04.04.1991
(51) Int. Cl.: A61K 38/00, A61K 39/395, A61K 35/58

(54) **INHIBITION OF ALLOGRAFT AND CONCORDANT XENOGRAFT REJECTION**
HEMMUNG DER ABSTOSSUNG BEI ALLOGENER UND CONCORDANTER XENOGENER TRANSPLANTATION
PREVENTION DU REJET D'UNE ALLOGREFFE ET D'UNE HETEROGREFFE CONCORDANTE

(30) Priority: 09.04.1990 GB 9007971
(43) Date of publication of application: 24.02.1993
(73) Proprietor: IMUTRAN LIMITED, London EC1A 2DY (GB)
(72) Inventor: WHITE, David James Graham, London EC1A 2DY (GB); VAN DEN BOGAERDE, Johan Beyers, London EC1A 2DY (GB)
(74) Representative: Sheard, Andrew Gregory
(86) International application number: GB9100522
(87) International publication number: WO9115221

(56) References cited:
- Chemical Abstracts, vol. 107, no. 1, 6 July 1987, Columbus, Ohio, US; H. Adachi et al.: "Effects of cyclo-sporine, aspirin, and cobra venom factor on discordant cardiac xenograft survival in rats", see page 36
- Dialog 6170074 Embase (Exerpta Medica), abst. 86165134; S.J. Knechtle et al.: "The effect of cyclosporine, total lymphoid irradiation, and cobra venom factor on hyperacute rejection"

## Description

This invention relates to the use of pharmaceutical formulations in the treatment of chronic rejection of allografts and rejection of concordant xenografts. The invention therefore relates to the promotion of graft survival, or prevention of graft loss, in a host.

Allografting is the transplantation of tissue between two members of the same species. Xenografting, which is the transplantation of tissues between two different species, has been divided by biological criteria into two subsets (Calne, *Transplant. Proc.* **II** 550-556 (1970)). The two subsets are concordant xenografts and discordant xenografts. The distinction between these two subsets is that a vascularised organ graft transplanted between discordant species is rejected hyperacutely, that is to say within a few minutes or hours of revascularisation. Between concordant species, hyperacute rejection of xenografts does not take place. Grafts will, however, be rejected, and often in an accelerated manner, but this will occur over several days and not within hours or minutes.

It is a generally held belief, although formal evidence for this is lacking, that between discordant species hyperacute rejection is a result of the action of naturally occurring antibodies against the donor species. Concordant grafts on the other hand survive at least for a short while because no such antibodies capable of causing their destruction are present. There are many examples in the literature to demonstrate this difference. Perhaps the most elegant are those of Hammer (in "Xenograft 25" (Mark A. Hardy, Ed) Elsevier, Amsterdam (1989)). Using always the dog as the recipient species, Hammer indicates the survival times of kidney grafts to be as follows:

| | |
|---|---|
| Dog-Dog | 11.4 days |
| Dingo-dog | 11.5 days |
| Wolf-dog | 19.4 days |
| Fox-dog | 6.5 days |
| Lion-dog | 10-24 hours |
| Tiger-dog | 10-24 hours |
| Cat-dog | 10-24 hours |
| Pig-dog | 10-30 minutes |

The last four interactions would on this basis be discordant xenografts, whereas the first four xenografts would be concordant. Hammer has equated these differences between discordant and concordant xenografts to the presence or absence, respectively, of cross-species haemagglutinating antibodies.

At an academic level, there are some isolated difficulties with what is essentially a working definition of concordant and discordant species relationships. As a practical matter, however, for the purposes of the present invention, two different species are taken to be related in a concordant fashion if there is no hyperacute rejection of a xenograft from one to the other. For the purposes of the present invention, therefore, species are concordantly related if, even though they would normally give rise to hyperacute rejection, they have nevertheless been subjected to a procedure, for example genetic manipulation, so as to prevent hyperacute rejection. International Patent Application PCT/GB90/01575 for example relates in specific embodiments to potential xenograft donor animals which have been transgenically modified to express recipient complement down regulators (such as decay accelerating factor (DAF) or membrane cofactor protein (MCP)). Organs from the donor animals are not hyperacutely rejected when grafted onto an animal of what would normally be regarded as a discordantly related species. For the purposes of this invention, therefore, such transgenic donor animals may be regarded as concordantly related to their intended target species.

Examples of species having a concordant relationship, as defined above, include hare-rabbit, hamster-rat and non-human primate-human, including, for example, baboon-human, chimpanzee-human and macaque-human.

The promotion of graft survival has received attention both in the field of xenografts (discordant and concordant) and allografts. Taking first suggestions that have been made for the promotion of discordant xenograft survival, Adachi *et al* (*Transplant. Proc.* **XIX** (1) 1145-1148 (1987)) have studied the survival of discordant cardiac xenografts in rats. Among the potential survival promoters studied were cyclosporin A, aspirin and cobra venom factor. Additionally, the combination of cyclosporin A and cobra venom factor was studied. Adachi *et al* report that "in our study, there was no statistically significant difference in mean survival time between the group treated with cyclosporin A and cobra venom factor and the group treated with cobra venom factor alone". Mean survival times were between 40 and 50 hours. Kemp *et al* (*Transplant. Proc.* **XIX** (6) 4471-4474 (1987)) also studied various potential discordant xenograft survival promoters in a study of rabbit kidney xenografts into cats. Candidate survival promoters included captopril, enalapril, nifedipine, prostacyclin, sodium chromoglycate, an azathioprine/corticosteroid combination, cyclosporin A and cobra venom factor. Hyperacute rejection was not prevented by any of these candidate drugs except for cobra venom factor which, the authors state, "had a striking effect, with prolongations in graft survival up to seven days".

Concordant xenograft rejection has been studied by Hardy *et al* (*Transplantation* **33** (3) 237-242 (1982)), who examined the effect of selective lymphoid irradiation (SLI) with palladium-109-haematoporphyrin combined with antilymphocyte globulin, a therapy which is primarily directed against T-cell-mediated rejection. Mean survival times were in the order of 12.5 days, compared with untreated controls which survived for 2.9 days on average. Homan *et al* (*Transplantation* **31** (3) 164-166 (1981)) studied the effect of cyclosporin A in the promotion of concordant xenograft survival. The best graft survival was for a medium of 21 days, compared to 2 days for untreated controls. Monden *et al* (*Transplantation* **43** (5) 745-746 (1987)) studied hamster-to-rat liver xenografts and found that cyclosporin A had no significant graft survival promoting effect when compared to untreated controls. Valdevere *et al* (*Transplant. Proc.* **XIX** (1) 1158-1159 (1987)) also studied hamster-to-rat liver xenografts and found a significant improvement in graft survival time, compared to controls, when cyclosporin A was administered in conjunction with splenectomy; survival was improved from an average of 3.6 days to an average of 17.6 days. Knechtle *et al* (*Transplant. Proc.* **XIX** (1) 1137-1139 (1987)), like the Hardy *et al* group referred to above, studied the effect of total lymphoid irradiation (TLI), but this time in conjunction with cyclosporin A therapy, on the promotion of concordant xenograft survival. They report that, in hamster-to-rat cardiac xenografts, cyclosporin A in high doses significantly prolonged survival, but only an occasional recipient had long-term graft function. TLI in combination with cyclosporin A significantly further promoted graft survival.

Conventional therapy against allograft rejection is to use cyclosporin A either on its own or in combination with steroids or azathioprin or both. In the opinion of some, the combination of triple therapies does not represent an improvement over therapy using cyclosporin A alone. These therapies are effective in the prevention and reversal of acute graft rejection, and probably retard the onset of chronic graft rejection, but appear to have little or not effect on the reversal of chronic rejection after onset.

Knechtle *et al* (*Heart Transplantation & Immunology* **IV** No 5: 541-545 (Sept/Oct 1985)) have analysed the effect in rats of cyclosporine, total lymphoid irradiation and cobra venom factor on hyperacute rejection. It was concluded that the addition of total lymphoid irradiation or cyclosporin to treatment with cobra venom factor did not result in longer survival than cobra venom factor alone.

The present invention seeks to promote the better survival of, or to rescue from rejection, concordant xenografts and/or allografts. The invention is based on the realisation that concordant xenograft survival, and also allograft survival, can be markedly enhanced by treatment with a combination of cyclosporin A and cobra venom factor, or by combinations of compounds having similar modes of action.

According to one aspect of the present invention, there is provided the use of a complement inhibitor and an immunosuppressant with cyclosporin A-like activity in the preparation of an agent or agents for promoting allograft or concordant xenograft survival or rescue from rejection; wherein said allograft survival or rescue from rejection is in respect of an allograft which is not hyperacutely rejected.

Pharmaceutical formulations which can be used in accordance with the invention therefore have two components, at least. A first component is a complement inhibitor. The complement inhibitor may either prevent the activation of complement or remove or deplete it, or one or more of its components. Preferably, the complement inhibitor will inhibit both the classical and alternative pathways of complement activation. It is therefore preferred that the complement inhibitor acts to block the pathway at C3, whether by inhibition or removal or depletion. It will be appreciated, therefore, that antibodies (for example monoclonal antibodies) against C3 may be used as the complement inhibitor in the present invention. Other useful complement inhibitors include soluble decay activating factor (DAF) and soluble membrane cofactor protein (MCP). One of the most preferred complement inhibitors is cobra venom factor.

Snake venoms are among the most complex of animal secretions, containing a vast number of substances with different pharmacological and biochemical activities. The presence in venoms of principles affecting complement has been known for almost a century. The action of the venom of the cobra (*Naja naja kaouthia*) on serum complement has been studied in some detail. The active factor is a glycoprotein with a molecular weight of 144kDa, which combines with serum factor B in the presence of magnesium ions. Factor B is then split into two components by the proteolytic action of serum factor D, creating Bb and Ba. The complex CoF-Bb, where CoF represent cobra venom factor, is a highly active C3- and C5-splitting enzyme or convertase. However, unlike normal C3- and C5-convertases, the CoF-Bb is resistant to the normal complement regulatory systems. Therefore, its action will continue, until all C3 has been converted, thus effectively depleting the individual of complement activity. Splitting of C5 releases C5a, which has anaphylactoid action; not all *Naja* cobra venom factors possess this C5 convertase activity, but this is not believed to be important for the purposes of the present invention. Inhibition of complement is not restricted to venoms of the *Naja* genus. Eggertsen *et al* (*Toxicon* **18** 87-96 (1980)) have studied venoms from snakes of the families Elapidae Viperidae and Crotalidae. Of 26 venoms studied, 22 have the ability to inhibit the activity of normal human complement. Analysis of the structure of cobra venom factor suggests that it is the equivalent of cobra C3 or a partially degraded product of it.

Cobra venom factor, and other snake venom factors, will generally not be administered in the present invention in their native form, because of the obvious problems of toxicity. In the absence of other measures to combat the toxicity problem, therefore, it will generally be necessary at least partially to purify the active factor from the crude venom. It is not necessary for cobra or other snake venom factor to be purified to homogeneity for use in the present invention, but clearly regulatory problems if nothing else would tend to prescribe the use of a substantially purified factor. Cobra venom factor, as a semi-purified preparation, can be obtained from chemical suppliers such as Sigma (Sigma Chemicals Limited, Poole, Dorset), who also supply crude cobra venom for in-house purification. An example of a preparative process for cobra venom factor from crude cobra venom is given in the examples.

It will be appreciated that, in addition to the use of natural cobra venom factor and other snake venom factors, analogues of such factors (including active fragments) can be used in this invention, as can synthetic factors including those produced by recombinant DNA technology. The characteristic of CoF that makes it suitable for use in the present invention is its ability to form a stable complex with factor Bb which is resistant to the normal breakdown mechanism. Other molecules having this characteristic are therefore also preferred for use in the invention.

A second component of pharmaceutical formulations which can be used in accordance with this invention is an immunosuppressant with cyclosporin A-like activity. Such compounds will be well known to those skilled in the art and include cyclosporins other than cyclosporin A, such as cyclosporin C and cyclosporin G. Eicosanoids also have cyclosporin A-like activity and include PGI₂ (prostacyclin), PGE₂, PGD₂, rapamycin and FK 506 (Fujisawa). Compound FK 506 is one of those that is preferred for use in the present invention, but the most preferred is cyclosporin A itself. Cyclosporin A is available from Sandoz under the trademark SANDIMUN.

A particularly preferred embodiment of the present invention uses a pharmaceutical formulation which comprises cobra venom factor and cyclosporin A, two of the most preferred individual compounds discussed above.

It will be appreciated that other ingredients may be present in pharmaceutical formulations for use in accordance with the invention. Excipients will usually be present, and the presence of other active ingredients is not excluded. More than one active ingredient of each category may be present.

It will be appreciated that the active ingredients do not necessarily have to be co-administered in a single formulation. A product containing a complement inhibitor and an immunosuppressant with cyclosporin A-like activity can be used as a combined preparation for simultaneous, separate or sequential use in promoting allograft or concordant xenograft survival or rescue from rejection.

A kit comprising a pharmaceutical preparation of a complement inhibitor and a pharmaceutical preparation of an immunosuppressant with cyclosporin A-like activity may be used.

Cyclosporin A and other immunosuppressants with cyclosporin A-like activity will usually be administered parenterally in the practice of the present invention. In such cases, the preparation containing the immunosuppressant will be sterile. However, cyclosporin A itself, and some other immunosuppressants, may be administered orally, in which case sterility is not essential. Cyclosporin A itself is only sparsely soluble in water and for practical purposes it is preferred to dissolve it in an oil, such as a vegetable oil. Olive oil has been found to be acceptable in practice. Dissolution in oil is only necessary when dealing with an immunosuppressant which is not soluble in water, and in general terms, the immunosuppressant may be dissolved in any appropriate physiologically acceptable carrier. In some cases, dissolution may not even be necessary. The concentration at which the immunosuppressant is prepared will of course depend on the nature of the immunosuppressant itself and the intended dose, which will generally be under the guidance of the clinician or physician. As a general guide, however, it has been found appropriate to prepare formulations of the cyclosporin at a concentration of from 1 to 100 mg/ml, for example from 1 to 10 mg/ml, typically about 5 mg/ml for parenteral purposes or 25 to 100 mg/ml, for example from 50 to 100 mg/ml, typically about 100 mg/ml for oral administration.

Complement inhibitors such as cobra venom factor will generally be given parenterally and for this purpose will generally be sterile. Cobra venom factor itself may be formulated in phosphate buffered saline (without azide) or any other suitable solvent or carrier. It may be that oral formulations can be developed. The concentration of complement inhibitor in a pharmaceutical formulation will again depend on the ultimate intended dose, which will be under the control of the clinician or physician. For guidance, however, a preparation of cobra venom factor in PBS can be made at a concentration ranging from 0.05 mg/ml up to the limit of solubility, which will be dependent on its purity. Compositions having a concentration up to 5 mg/ml may be useful in practice, for example those having a concentration of from 0.1 to 2 mg/ml, for example about 0.5 mg/ml.

Because cyclosporin A is essentially not soluble in aqueous media, whereas cobra venom factor is, these two preferred active ingredients will generally be formulated separately and administered separately in practice. This is not in principle essential, however, as it may be possible to formulate an appropriate emulsion of the two active ingredients, and different combinations of active ingredients may be soluble in a common solvent or at least suspendable in a common carrier.

Reference has been made above to parenteral injection. The preferred site of injection is muscle, because of the depot effect resulting from an intramuscular injection. Intravenous injection, however, may be appropriate in some circumstances and subcutaneous injection may have some if not all of the advantages of intramuscular injection. Intraperitoneal injection could be used if circumstances dictate.

It may be preferable in many circumstances to pre-administer some or all of the active ingredients useful in this invention before graft surgery takes place. It should also be noted that a primary advantage of the present invention as it relates to allografts is that the combination of active ingredients useful in the present invention would be useful for preventing chronic rejection or managing chronic rejection (ie rescuing from rejection) after its onset; this usually happens many years post-transplant.

Allograft recipients suffering from chronic graft rejection, or at risk from chronic graft rejection, may well be receiving immunosuppression with cyclosporin A or another immunosuppressant having cyclosporin A-like activity. For the purposes of this invention, in order to treat or prevent chronic graft rejection in such patients it is necessary only to add complement inhibitor therapy. According to one aspect of the invention, there is provided the use of a complement inhibitor in the preparation of an agent for promoting allograft or concordant xenograft survival, or rescue of such a graft from rejection, in a graft recipient being treated with an immunosuppressant with cyclosporin A-like activity. The invention therefore has application in a method of promoting allograft or concordant xenograft survival and/or of rescuing such a graft from rejection, in a host, the method comprising administering to a graft recipient or an intended graft recipient, being treated with an immunosuppressant with cyclosporin A-like activity, a complement inhibitor.

The immunosuppressant with cyclosporin A-like activity will be administered at any suitable effective but non-toxic level, having regard to the species being treated and the organ being transplanted. The upper limit of cyclosporin A tolerance in humans is determined by its nephrotoxicity and the age of the recipient. 20 mg/kg per day may be a safe upper limit for treating humans in many cases, although other species may well have higher tolerances. Cyclosporin A is effective at low doses, and so the lowest amount that may be administered will simply be the lowest amount that gives an effective response. Amounts of 1 or 2 mg/kg per day have in some cases found to be effective. As a general guide, between 5 and 10 mg/kg per day can be administered. If administered parenterally, cyclosporin A may be given on alternate days, in which case the dose would be doubled. For the preferred, oral route, however, daily dosage will be the regimen of choice; more particularly cyclosporin A may be administered twice or more frequently per day to give a total daily dose in the order of 5 to 10 mg/kg, for preference. Other immunosuppressants with cyclosporin A-like activity will be administered at effective but non-toxic doses, as will be determined by the clinician or physician.

The complement inhibitor, such as cobra venom factor, will as a guiding principle be administered in an amount which effectively inhibits complement activity, as determined by the complement haemolysis₅₀ (CH₅₀) assay. The safe upper limit for the administration of cobra venom factor has not been determined, but doses of 2 mg/kg do not appear to be toxic. The minimum dosage of cobra venom factor is species dependent and can readily be determined by the CH₅₀ assay. Daily doses of between 0.01 and 0.1 or 0.2 mg/kg have been found to be effective for cobra venom factor. Effective and non-toxic dosages of other complement inhibitors can also readily be determined by those skilled in the art, for example, using the CH₅₀ assay.

Cobra venom factor takes about 6 hours for its initial activity to take effect after intramuscular administration. Once the initial activity is observed, it can last for more than 1 day, for example 2 days, and so daily administration of cobra venom factor may not be necessary. In fact, it is preferred to administer cobra venom factor on alternate days, in which case the preferred daily dosages given above will be doubled on the day of administration. In practice, it has been found that a particularly suitable dosage regimen is to administer 0.4 mg/kg intramuscularly on alternate days.

Cyclosporin A or other immunosuppressant administration will usually be continuous. Cobra venom factor or other complement inhibitor administration will generally not be continuous, but may be prolonged; even this may not in practice be essential, as short term therapy may be all that is needed for the treatment for, or rescue from rejection of, concordant xenografts.

The invention will now be illustrated by reference to the following preparation and examples. The examples refer to the accompanying drawings, in which:
FIGURE 1 is a graph of hamster heart xenograft survival in rats, with percentage survival being plotted against days post transplant; and
FIGURE 2 is a graph of hamster heart xenograft survival in cyclophosphamide treated rats; the graph otherwise corresponds to Figure 1.

### Preparation

This preparation gives a protocol for preparing cobra venom factor from crude cobra venom.

1g of venom of *Naja naja kaouthia* (Sigma, Product No. V-9125, Lots Nos. 116F-0398 and 58F-0565) was dissolved in 60 ml of degassed sodium phosphate buffer (0.01M) at pH 7.5 and pumped onto a 90cm x 26mm (internal diameter) column of DEAE cellulose (DE52, Whatman) and equilibrated with the same buffer. The column was then eluted at 43.0 ml per hour with the starting buffer until the A₂₈₀ absorbence of the effluent returned to 0. A linear 3 litre:3 litre gradient of starting buffer and limit buffer (0.01M sodium phosphate, pH 7.5, containing 0.5M sodium chloride) was then pumped on at 40.0 ml per hour and 7.0 ml fractions were collected. Fractions were tested for anti-complementarity, and the positive fractions were pooled and concentrated on CX-10 ultrafilters.

The whole of the cobra venom factor pool was pumped onto a 90cm x 26mm (internal diameter) column of Sephadex® G200 (fine) (Pharmacia GB Limited, London), equilibrated with PBS (no azide) and eluted with PBS at 36 ml per hour. 6 ml fractions were collected and assayed for anti-complementarity activity. The cobra venom factor fractions (ie the active fractions) were pooled, especially on the descending limb of the peak, concentrated on CX-10 ultrafilters, partitioned into aliquots and stored frozen at -20^{o}C ready for use.

The anti-complementary activity of cobra venom factor referred to above can be measured by the following method. 20 µl fractions were incubated with 20 µl of fresh human serum for 20 minutes at 37^{o}C. 5 µl amounts were then added to wells in a test plate, made from:
5.0 ml 2% aqueous agarose;
4.5 ml double strength PBS including 20mM EDTA;
0.5 ml 10% washed guinea pig erythrocytes; and
0.5 ml fresh normal human serum.

The plate was then incubated overnight at +4^{o}C. Positives are shown by clear zones of lysis after incubation.

### Example 1 (Comparison Example)

In this example, the rats are DA rats from Banting and Kingdom, and the hamsters are Syrian hamsters, also from Banting and Kingdom. Following the method of Heron (*Acta Pathol. Microbiol. Scand.* **79** 366-372 (1971)), hamster hearts were grafted into the necks of the rats. The aorta was joined to the carotid artery using the cuff technique described by Heron. Similarly, the pulmonary artery was joined to the jugular vein. All other cardiac vessels were ligated. Hearts began beating with a few minutes of removal of clamps. Skin was then closed over the implanted heart, which was monitored daily by external palpation.

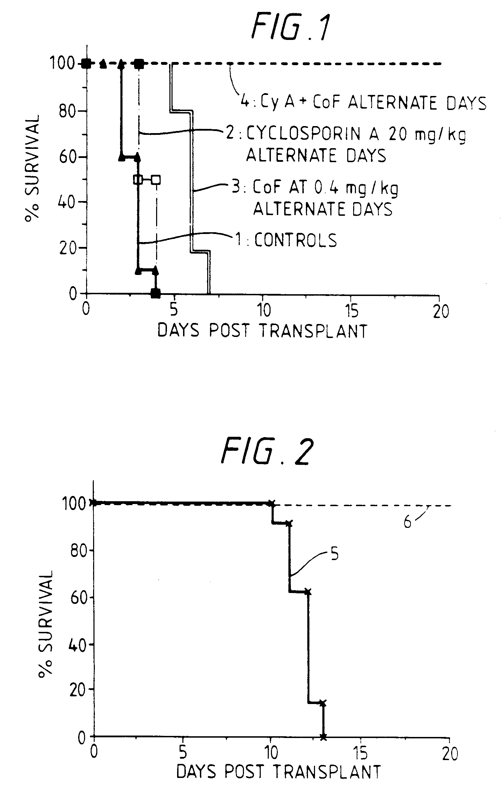

Heart graft survival data are shown in Figure 1 and are represented by line 1. It can be seen that the median survival rate of the grafts was 3 days.

### Example 2 (Comparison Example)

The procedure of Example 1 was repeated, except that cyclosporin A was administered at a dosage of 20 mg/kg per alternate day. The cyclosporin A was administered as a 20 mg/ml preparation in olive oil and injected intramuscularly, with the first dose being given at the time of transplantation. Injections were given on alternate days thereafter. Graft survival data are again shown in Figure 1: line 2 gives the results from this example. The median graft survival rate is 3 days.

### Example 3 (Comparison Example)

The procedure of Example 1 was repeated, except that cobra venom factor was administered at a dosage of 0.4 mg/kg per alternate day. The cobra venom factor was administered as a 0.5 mg/ml solution in azide-free PBS. The first injection was made intramuscularly at the time of transplantation, and subsequent intramuscular injections were made on alternate days thereafter. The graft rejection data are again presented in Figure 1. The results are shown on line 3, which indicates that the median survival rate for the grafts is 6 days.

### Example 4

The procedure of Example 1 was repeated, except that both cyclosporin A and cobra venom factor were administered to the recipient. Cyclosporin A was administered at a dose of 20 mg/kg on alternate days, so that the conditions for cyclosporin administration were the same as in Example 2. The cobra venom factor was administered under the same conditions as described in Example 3. Both the cyclosporin A and the cobra venom factor were first administered at the time of transplantation. Graft survival data are again shown in the figure. In this example, no graft rejection is observed, as can be seen from line 4 of Figure 1. At present, median graft survival is in excess of 50 days.

### Example 5

The procedure of Example 4 was repeated, except that cobra venom factor administration was discontinued after 28 days post transplant. Median graft survival was greater than 100 days. Two animals were sacrificed at 103 and 105 days and histological examination of the graft showed normal cardiac architecture. A remaining rat has survived with a beating hamster heart for more than 5 months so far.

### Example 6 (Comparison Example)

20 rats were given hamster hearts by the protocol described in Example 1. They were treated with cyclosporin A as described in Example 2, and in addition they were given ENDOXAN cyclophosphamide i.p. (20 mg/kg) one day pre-transplant (day-1) and on day 2 post-transplant. (The word ENDOXAN is a trade mark for cyclophosphamide, which is an inhibitor of antibody production.) The hamster hearts were rejected as shown in line 5 of Figure 2. The observed delayed rejection, in which the delay was a result of the short cyclophosphamide therapy, simulates a delayed xenograft rejection of the type that would result from inadequate immunosuppressive protocols.

### Example 7

Five rats were treated as in Example 6, but additionally were treated with cobra venom factor as in Example 4 from day 7. Treatment with cobra venom factor lasted for 14 days. All animals were rescued from rejection, as can be seen from line 6 of Figure 2.

## Claims

1. The use of a complement inhibitor and an immunosuppressant with cyclosporin A-like activity in the preparation of an agent or agents for promoting allograft or concordant xenograft survival or rescue from rejection; wherein said allograft survival or rescue from rejection is in respect of an allograft which is not hyperacutely rejected.

2. The use of a complement inhibitor in the preparation of an agent or agents for promoting allograft or concordant xenograft survival, or rescue from rejection, in a graft recipient being treated with an immunosuppressant with cyclosporin A-like activity, wherein said allograft survival or rescue from rejection is in respect of an allograft which is not hyperacutely rejected.

3. The use as claimed in claim 1 or claim 2, wherein the complement inhibitor inhibits both the classical and alternative pathways of complement activation.

4. The use as claimed in any preceding claim, wherein the complement inhibitor comprises MCP, DAF or an antibody against C3.

5. The use as claimed in claim 1 or claim 2, wherein the complement inhibitor comprises a molecule which forms a stable complex with factor Bb, wherein the complex is resistant to normal breakdown mechanisms.

6. The use as claimed in any of claims 1 to 3, wherein the complement inhibitor comprises cobra venom factor.

7. The use as claimed in any preceding claim, wherein the immunosuppressant with cyclosporin A-like activity is a cyclosporin or an eicosanoid.

8. The use as claimed in claim 7, wherein the immunosuppressant is cyclosporin A.

9. The use as claimed in claim 7, wherein the immunosuppressant is FK 506.

10. The use according to any preceding claim, wherein the complement inhibitor and the immunosuppressant with cyclosporin A-like activity are for simultaneous, separate or sequential administration.

11. The use according to any preceding claim, wherein the complement inhibitor and the immunosuppressant with cyclosporin A-like activity are provided in kit form.

## Patentansprüche

1. Verwendung eines Komplementinhibitors und eines Immunsuppressivums mit Cyclosporin-A-ähnlicher Wirksamkeit bei der Herstellung eines Mittels oder von Mitteln zur Förderung des Überlebens eines allogenen Transplantats oder eines konkordanten xenogenen Transplantats oder zur Verhinderung einer Abstoßung eines solchen; wobei sich das Überleben oder die Verhinderung der Abstoßung des allogenen Transplantats auf ein allogenes Transplantat bezieht, das nicht hyperakut abgestoßen wird.

2. Verwendung eines Komplementinhibitors bei der Herstellung eines Mittels oder von Mitteln zur Förderung des Überlebens eines allogenen Transplantats oder eines konkordanten xenogenen Transplantats oder zur Verhinderung einer Abstoßung eines solchen in einem Transplantatempfänger, der mit einem Immunsuppressivum mit Cyclosporin-A-ähnlicher Wirksamkeit behandelt wird, wobei sich das Überleben oder die Verhinderung der Abstoßung des allogenen Transplantats auf ein allogenes Transplantat bezieht, das nicht hyperakut abgestoßen wird.

3. Verwendung nach Anspruch 1 oder Anspruch 2, wobei der Komplementinhibitor sowohl die klassischen als auch die alternativen Wege der Komplementaktivierung hemmt.

4. Verwendung nach irgendeinem der vorhergehenden Ansprüche, wobei der Komplementinhibitor MCP, DAF oder einen Antikörper gegen C3 umfaßt.

5. Verwendung nach Anspruch 1 oder Anspruch 2, wobei der Komplementinhibitor ein Molekül umfaßt, das einen stabilen Komplex mit dem Faktor Bb bildet, wobei der Komplex gegen normale Abbaumechanismen resistent ist.

6. Verwendung nach irgendeinem der Ansprüche 1 bis 3, wobei der Komplementinhibitor den Kobragift-Faktor umfaßt.

7. Verwendung nach irgendeinem der vorhergehenden Ansprüche, wobei das Immunsuppressivum mit Cyclosporin-A-ähnlicher Wirksamkeit ein Cyclosporin oder ein Eicosanoid ist.

8. Verwendung nach Anspruch 7, wobei das Immunsuppressivum Cyclosporin A ist.

9. Verwendung nach Anspruch 7, wobei das Immunsuppressivum FK 506 ist.

10. Verwendung nach irgendeinem der vorhergehenden Ansprüche, wobei der Komplementinhibitor und das Immunsuppressivum mit Cyclosporin-A-ähnlicher Wirksamkeit zur gleichzeitigen, gesonderten oder aufeinanderfolgenden Verabreichung vorgesehen sind.

11. Verwendung nach irgendeinem der vorhergehenden Ansprüche, wobei der Komplementinhibitor und das Immunsuppressivum mit Cyclosporin-A-ähnlicher Wirksamkeit in Form eines Kits zur Verfügung gestellt werden.

## Revendications

1. Utilisation d'un inhibiteur du complément et d'un immunosuppresseur avec une activité analogue à celle de la cyclosporine A dans la préparation d'un agent ou d'agents pour favoriser la survie d'allogreffe ou d'hétérogreffe concordante ou prévenir le rejet; dans laquelle la survie d'allogreffe ou la prévention du rejet concerne une allogreffe qui n'est pas rejetée de manière hyperaiguë.

2. Utilisation d'un inhibiteur du complément dans la préparation d'un agent ou d'agents pour favoriser la survie d'allogreffe ou d'hétérogreffe concordante, ou prévenir le rejet, chez un receveur de greffe traité au moyen d'un immunosuppresseur avec une activité analogue à celle de la cyclosporine A, dans laquelle la survie d'allogreffe ou la prévention du rejet concerne une allogreffe qui n'est pas rejetée de manière hyperaiguë.

3. Utilisation selon la revendication 1 ou 2, dans laquelle l'inhibiteur du complément inhibe à la fois les voies classique et alternative d'activation du complément.

4. Utilisation selon l'une quelconque des revendications précédentes, dans laquelle l'inhibiteur du complément comprend le MCP, le DAF ou un anticorps contre C3.

5. Utilisation selon la revendication 1 ou 2, dans laquelle l'inhibiteur du complément comprend une molécule qui forme un complexe stable avec le facteur Bb, où le complexe est résistant aux mécanismes normaux de dégradation.

6. Utilisation selon l'une quelconque des revendications 1 à 3, dans laquelle l'inhibiteur du complément comprend le facteur du venin de cobra.

7. Utilisation selon l'une quelconque des revendications précédentes, dans laquelle l'immunosuppresseur avec une activité analogue à celle de la cyclosporine A est une cyclosporine ou un eicosanoïde.

8. Utilisation selon la revendication 7, dans laquelle l'immunosuppresseur est la cyclosporine A.

9. Utilisation selon la revendication 7, dans laquelle l'immunosuppresseur est le FK 506.

10. Utilisation selon l'une quelconque des revendications précédentes, dans laquelle l'inhibiteur du complément et l'immunosuppresseur avec une activité analogue à celle de la cyclosporine A sont destinés à une administration simultanée, séparée ou séquentielle.

11. Utilisation selon l'une quelconque des revendications précédentes, dans laquelle l'inhibiteur du complément et l'immunosuppresseur avec une activité analogue à celle de la cyclosporine A sont fournis sous forme de trousse.
